# EUROPEAN PATENT SPECIFICATION

(11) **EP 0 874 623 B1**
(45) Date of publication and mention of the grant of the patent: **17.09.2003**
(21) Application number: 96922144.9
(22) Date of filing: 03.07.1996
(51) Int. Cl.: A61K 9/70, A61L 15/44

(54) **WATER ACTIVATABLE ADHESIVE MATRIX CONTAINING AMETHOCAINE**
DURCH WASSER AKTIVIERBARE AMETHOCAIN ENTHALTENDE KLEBEMATRIX
MATRICE ADHESIVE ACTIVABLE PAR L'EAU CONTENANT DE L'AMETHOCAINE

(30) Priority: 06.07.1995 GB 9513742
(43) Date of publication of application: 04.11.1998
(73) Proprietor: Smith & Nephew PLC, London WC2N 6LA (GB)
(72) Inventor: McCAFFERTY, Dermot, Belfast BT8 4YR (GB); WOOLFSON, Aaron, David, Belfast BT9 6TF (GB)
(86) International application number: GB9601594
(87) International publication number: WO97002003

(56) References cited:
- EP-A- 0 200 508
- EP-A- 0 223 524
- WO-A-88/09169
- WO-A-92/15289
- WO-A-92/16202
- GB-A- 1 108 837
- PATENT ABSTRACTS OF JAPAN vol. 14, no. 037 (C-680), 24 January 1990 & JP 01 272251 A (TEIKOKU SEIYAKU KK), 31 October 1989, & DATABASE WPI Section Ch, Week 8949 Derwent Publications Ltd., London, GB; Class A12, AN 89-361691 & JP 01 272 521 (TEIKOKU SEIYAKU KK) , 31 October 1989
- J. PHARM. PHARMACOL., vol. 36, 1984, pages 295-299, XP002020132 SMART J. D. ET AL: "An In-vitro Investigation of Mucosa-adhesive Materials for use in controlled drug delivery "
- AM. PHARM. ASSOC. AND PHARM. SOC. OF GREAT BRITAIN: "Handbook of Pharmaceutical Excipients" 1988 , AMERICAN PHARMACEUTICAL ASSOC. , WASHINGTON XP002020922 153140 see page 47, paragraph 11. see page 48, paragraph 17. see page 132, paragraph 11. see page 133, paragraph 17.

## Description

This invention relates to pharmaceutical compositions useful for anaesthetising intact human skin. More particularly, it relates to film-forming compositions containing amethocaine (2-dimethyl aminoethyl para-aminobenzoate).

Topical compositions containing amethocaine are known in the art to provide effective local anaesthesia of intact, healthy skin. The local anaesthetic must be presented to the skin surface substantially in its uncharged, lipophilic form such that it penetrates the outer, lipophilic skin barrier, the stratum corneum, in order to reach and desensitise the underlying pain receptors within the skin structure.

British Patent No. 2,163,956 discloses aqueous gel compositions containing amethocaine in dispersion as its free base. Under these conditions, the melting point of amethocaine base is lowered from approximately 41°C to approximately 30°C. Such compositions therefore undergo a phase change from solid to an oily liquid drug dispersion when applied to human skin, the temperature of which is typically 32°C. This phase change is an integral part of the mechanism of action of said compositions, as disclosed in Woolfson, A.D. and McCafferty, D.F. Percutaneous local anaesthesia: Drug release characteristics of the amethocaine phase-change system. International Journal of Pharmaceutics, volume 94, pages 75-80 (1993).

Amethocaine gel compositions of this type produce sufficient local anaesthesia of intact healthy skin to render it insensitive to needle penetration challenge and other minor surface surgical procedures. Such compositions suffer from the following disadvantages in respect of ease of use and susceptibility to chemical degradation of the active ingredient:
(1) Since the compositions disclosed are aqueous gels, they require to be spread over the intact skin surface at the site of application. This results in a variable area of skin being covered with each application.
(2) The process of spreading the gel over the skin is inconvenient and messy.
(3) Such gels do not readily lend themselves to self-application, particularly when large areas are to be covered, for example, in the anaesthetising of an area of skin to be harvested for subsequent use in split-skin grafting.
(4) The gel compositions must be covered with a suitable separate dressing after applying said gel to the skin site. This results in an unsightly area on the skin during treatment, with a risk of the gel oozing from the sides of the covering dressing and contacting clothing or other areas of skin.

International Patent Application No. PCT/GB92/00170 discloses further gel compositions containing amethocaine and suitable for percutaneous anaesthetic use on intact healthy skin. Said compositions claim enhanced stability over those disclosed in British Patent No. 2,163,956 by means of providing a salt component such that the solubility of amethocaine base in the aqueous gel vehicle is reduced, with a concomitant reduction in the rate of alkaline hydrolysis of the solution component of the drug. The pH of the drug dispersion is also preferably maintained below 8 by generating the free base form of amethocaine from its salt, amethocaine hydrochloride, in situ.

United States Patent No. 1,108,837, discloses a sheet-like material of a water-soluble, film-forming compound or composition having incorporated therein a local anaesthetic agent, most typically of the lidocaine type but also incorporating the use of ester type local anaesthetics such as amethocaine. Said film-forming compound or composition holds the local anaesthetic in solution within the matrix thus formed, or as an extremely fine or colloidal dispersion. It is an aspect of this invention that the sheet-like local anaesthetic composition is intended as an alternative to infiltration of the active local anaesthetic ingredient by use of an injection needle. There is no teaching that said compositions can anaesthetise intact skin via the percutaneous route, the compositions of the invention being intended for application to mucosal epithelia or to broken skin, where the stratum corneum barrier is not present. In a further aspect to the invention, it is disclosed that said film forming compound should be water-soluble, the release of the active agent being dependent on initial dissolution of the sheet-like material.

British Patent Application No. 9219079.2 discloses unit-dose film compositions of amethocaine suitable for producing percutaneous anaesthesia of intact human skin. The disclosure encompasses a hydrophilic gel containing amethocaine that is dried to a film consistency. The film must be thoroughly and directly wetted in order to activate the amethocaine phase change.

Adhesives suitable for securing non or low adherent films to human skin are known in the art. Such adhesives are of the pressure-sensitive type. Pressure-sensitive adhesives may be produced conventionally by compounding an elastomer with a tackifying resin, or alternatively by using polymers which are inherently pressure-sensitive such as the polyacrylates and polyvinylether adhesives. A disadvantage of pressure-sensitive adhesives is that adhesion is lost or substantially reduced when the substrate and/or the environment is wet. Loss of adhesion in these cases is due to the adhesive absorbing moisture and swelling. Consequently, the film becomes detached from the skin surface. In addition, water effectively acts as a lubricant between skin and pressure-sensitive adhesive, preventing full bond strength from being developed and leading to immediate or rapid bond failure. The properties of conventional, pressure-sensitive adhesives known in the art may be found in Satas, D (Editor): *Handbook of Pressure-Sensitive Adhesive Technology,* Van Nostrand Reinhold, New York, 1982. It will be apparent to those skilled in the art, therefore, that percutaneous anaesthetic compositions of amethocaine that require the presence of water to activate the solid-liquid phase change essential for drug release are essentially incompatible with the use of such pressure-sensitive adhesives for the purpose of securing the amethocaine compositions to intact, healthy human skin.

Water-activated adhesives have been known in the art for some time. These so-called 'wet-stick' adhesives or bioadhesives have been used in the formulation of novel drug delivery systems. Numerous examples may be found in Park *et al*. (1987) Bioadhesive Hydrogels. In: Peppas (ed) *Hydrogels in Medicine and Pharmacy,* Vol. III. CRC Press, Boca Raton, Florida and also in Duchene *et al*. Pharmaceutical and Biomedical Aspects of Bioadhesive Systems for Drug Administration, *Drug Development and Industrial Pharmacy,* Volume 14, pages 283-318 (1988).

Bioadhesive films for drug delivery applications have been known in the art for some time. Such drug-loaded films are designed primarily for application to wet mucosal epithelia rather than to intact healthy skin. In such cases, the process of adhesion involves interaction with a covering layer of mucin. For skin adhesion, mucin is not present and the adhesion process is substantially different. Conventionally , bioadhesives have the disadvantages of requiring water activation compared to the pressure-sensitive type. Aesthetically unacceptable tacky residues often remain when compositions having acceptable skin adhesion are subsequently removed from the skin site. Such compositions tend to retard drug release through their high micro-viscosity.

Bioadhesive compositions containing active medicaments, including local anaesthetics for the prevention of cutaneous pain, are disclosed in International Patent Application No. PCT/US92/01730 to Mantelle. Such compositions require the incorporation of a non-aqueous solvent for the medicaments such that said medicaments are not present in crystalline form. Furthermore, it is stated that, for local anaesthetics, it is preferable to include both their water-soluble salt and non water-soluble free base forms in the compositions. Such teaching is contrary to the known and unique mode of action of the amethocaine phase change system for percutaneous local anaesthesia, as disclosed in Woolfson, A.D. and McCafferty, D.F. Percutaneous local anaesthesia: Drug release characteristics of the amethocaine phase-change system, International Journal of Pharmaceutics, volume 94, pages 75-80 (1993). It is a prerequisite of this system that any solvent other than water is excluded and that amethocaine is substantially present as a solid crystalline dispersion in an aqueous medium in order to facilitate the solid-to-liquid phase change. Hereinafter such 'wet-stick' adhesives or bioadhesives shall be referred to as water activatable adhesives.

International Patent Application No.WO-A-8809169 describes amethocaine compositions in a solid matrix, mainly, hydrophilic synthetic plastic material films which can be applied directly to the skin. However, the films do not possess any adhesive properties.

Japanese Patent Application No.JP-A-1272521 describes the incorporation of, for example, narcotic materials into adhesive layers, but does not describe the use of either "wet-stick" adhesives or water activatable physiologically active components.

International Patent Application No. WO 92/16202 describes a percutaneous anaesthetic delivery system comprising a skin conformable backing layer, an amethocaine bearing material and a layer having little or no tendency to absorb amethocaine intermediate the backing layer and a layer of amethocaine bearing material. The backing layer may be adhesive coated such that it may be adhered to the skin of a patient but there is no mention of the use of "wet-stick" adhesives.

European Patent Application No.EP-A-0223524 describes the use of polycarboxylic acid and/or polycarboxylic acid anhydride to produce a film-like adhesive material which may have a pharmaceutical drug dispersed therein. There is no mention of the use of amethocaine and/or the use of "wet-stick" adhesives.

Conventional prior art formulations of amethocaine may also suffer from the disadvantage that they cannot be presented as a unit dose composition in such a manner that an identical amount of the active ingredient is applied at every application. Unit dose compositions are known in the art to offer benefits in respect of patient compliance and patient safety.

Accordingly, it is a novel aspect of the present invention to provide an amethocaine composition in which the amethocaine is in a crystalline free base form and is dispersed in a water activatable adhesive matrix capable of adhering to wet intact healthy human skin, which comprises at least one film forming adhesive polymer, and the composition is provided with a water impermeable backing layer laminated to the amethocaine composition by means of an intermediate transfer adhesive, characterised in that
a) the polymer is a copolymer of maleic anhydride and methyl vinyl ether, and
b) the intermediate transfer adhesive is a pressure sensitive adhesive.

The compositions of the invention are generally themselves non-adhesive prior to water activation of the water activatable adhesive matrix. Such compositions are generally in the form of a substantially dry film or substantially dry film-like form. By the term substantially dry we mean dried sufficiently so that the phase change undergone by amethocaine in the presence of water is not undergone.

The compositions of the invention will generally adhere directly to wet, intact healthy human skin and are capable of producing effective percutaneous anaesthesia of the treated site. The water activatable adhesive matrix is preferably not water-soluble. In use, either the skin of the patient or the amethocaine composition of the invention itself (or both) may be wetted prior to the application of the composition.

It is a further aspect of the present invention that said amethocaine compositions may be activated prior to use by the presence of water on the skin, such activation generating, simultaneously, the amethocaine phase change, an essential prerequisite for drug release and consequent effective clinical performance, and sufficient adhesion in the film composition that it may be securely attached to the required intact, healthy skin site. Alternatively the composition itself may be wetted prior to application to the skin.

It is a still further aspect of the present invention that said amethocaine compositions contain at least one film forming water activatable adhesive polymer. Such water activatable adhesive polymers should be present in a concentration less than that which would retard drug release from the water-activated composition, one or more substantially non water activatable adhesive polymer components. The purpose of the substantially non water activatable adhesive polymer component is to enhance film integrity without affecting drug release or adhesion, to enable location of the water activatable adhesive layer during a manufacturing coating procedure and to aid complete wetting of the amethocaine composition in use, thus simultaneously activating the adhesion and drug release mechanisms.

Said amethocaine compositions may remain securely located in place at the skin site by means of their water activated adhesion but are capable of being removed from the skin with ease by a peeling motion, such removal resulting in a minimum, water-washable and anaesthetically acceptable residue being left at the site.

It is a further additional aspect of the present invention that said amethocaine compositions are provided with a water-impermeable backing layer. The water impermeable backing layer in conjunction with the amethocaine composition form an integrated percutaneous anaesthetic patch delivery system. The purpose of said backing layer is, inter alia, when the system is in use to isolate the water-activated amethocaine composition from the environment during clinical use and to prevent the amethocaine composition from drying out. Said backing layer must therefore be impervious to penetration by amethocaine in order to ensure that, during storage, the effective amount of amethocaine dispersed in the water activatable adhesive matrix is not reduced by gradual amethocaine migration into the backing layer.

It is a still further additional aspect of the present invention that said backing layer may be laminated to the amethocaine composition. For example the backing layer and the amethocaine composition may be laminated by means of an intermediate transfer adhesive. The intermediate transfer adhesive may be a pressure sensitive adhesive. Said transfer adhesive not being exposed to the skin but forming an integral part of the overall patch structure.

It is a yet still further additional aspect of the present invention that said integrated percutaneous anaesthetic patch compositions are highly flexible and are substantially thin or ultra-thin such that they can readily conform to irregular skin surfaces. Also, the patch can be produced in sheets of various shapes/sizes and cut before use for areas eg around the eye or ear.

It will be apparent to those skilled in the art that the invention encompasses a range of water activatable adhesive polymers whose properties conform to the specific requirements of the invention, together with a range of substantially non water activatable adhesive polymers whose properties also conform to the requirements of the invention. It will also be apparent that the invention further encompasses the use of film modifying components known in the art such as pharmaceutically acceptable plasticisers.

A particularly preferred water activatable adhesive polymer of the invention is a copolymer of maleic anhydride and methyl vinyl ether, available commercially in a range of grades under the trade name Gantrez (Trade Mark) from Gaf Corporation. Particularly preferred substantially non water activatable adhesive polymer components of the invention are hydroxyethylcellulose or sodium carboxymethylcellulose, eg. cross-linked sodium carboxymethylcellulose. A particularly preferred pharmaceutically acceptable plasticiser is glycerin.

Therefore, according to the invention we provide, an amethocaine composition in which the amethocaine is in a crystalline free base form and is dispersed in a matrix comprising a maleic anhydride and methyl vinyl ether copolymer and hydroxyethylcellulose.

The composition according to the invention may contain between 1 and 5% w/w, and preferably between 1.5 and 2.5% w/w of the substantially water activatable adhesive polymer component. The compositions may also contain between 1 and 5% w/w, and preferably between 1.5 and 2.5% w/w of the non water activatable adhesive polymer component. Additionally, the compositions may optionally contain between 0.5 and 2% w/w of a pharmaceutically acceptable plasticiser. The compositions may also contain between 0.5% and 5% w/w, but preferably between 1 and 2.5% w/w of amethocaine base. It will be apparent to those skilled in the art that the pH of the final film-like composition should be such that there is sufficient amethocaine present in the free base form to allow effective penetration of the skin barrier structure.

According to a further feature of the invention we provide a method of manufacturing an amethocaine composition as hereinbefore described which comprises;
adjusting the pH of an aqueous dispersion of a water activatable adhesive polymer to pH greater than 7;
adding amethocaine free base to the buffered dispersion; and
drying the amethocaine composition.

We also provide the use of amethocaine, eg. amethocaine free base, in the manufacture of an amethocaine composition as hereinbefore described.

We further provide a method of percutaneous anaesthesia which comprises wetting an amethocaine composition or a patch system as hereinbefore described and subsequently adhering the composition or patch to the skin of a patient.

Alternatively the method of percutaneous anaesthesia may comprise first wetting the skin of a patient and subsequently adhering the amethocaine composition or patch as hereinbefore described to the wetted region of the patient's skin.

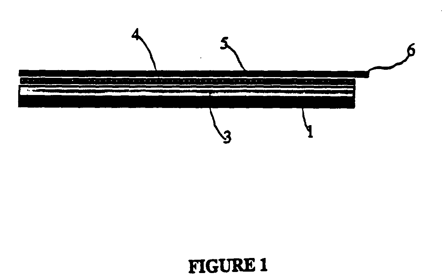

The embodiment of the invention will now be illustrated by reference to Figure 1 which is a cross-section of a patch according to the invention.

With reference to Figure 1, a patch (2) comprises a release liner (1) coated with a dry amethocaine gel film (3) as hereinbefore described. The dry film (3) is covered with a backing layer (5) of a metalised foil strip, which backing layer is optionally provided with a pharmaceutical grade transfer adhesive (4) on the dry film (3) side of the backing layer (5). The backing layer (5) extends beyond the edge (7) of the dry film (3) to provide a peel strip (6).

### Example 1

### Stage 1: Gel Preparation

Gantrez (Trade Mark) AN-139 co-polymer was added to a sufficient quantity of preheated water (95-99°C) to produce a final polymer concentration of 2% w/w. The water was stirred at a speed sufficient to produce a rapidly swirling vortex and the co-polymer was sifted slowly into the vortex so that the powder was wetted and dispersed. Temperature was maintained by the use of a water bath or other suitable heating mechanism.

The slurry, maintained at 95-99°C, was stirred at high speed until complete dissolution was achieved, indicated by a decrease in viscosity and a change in appearance from milky white to transparent. Any water lost by evaporation was replaced at room temperature with gentle stirring.

The resulting Gantrez (Trade Mark) AN-139 solution had a pH of approximately 1.5. The above reaction took about 20 minutes to completion.

The solution was allowed to cool to room temperature and the pH was then adjusted to pH9 with 30% sodium hydroxide (3.1 mls in 100g of solution), added slowly and dropwise with continual stirring.

The solution was rapidly stirred while hydroxyethylcellulose (Natrosol (Trade Mark) 250 HHX-Pharm from Aqualon) was added slowly in sufficient quantity until complete solution was achieved to yield a final concentration of 1.5% w/w. Glycerin was then added in sufficient quantity to yield a final concentration of 1% w/w.

Amethocaine base (sufficient to yield a final concentration of 1% w/w) was then added to the solution and, by gently heating the reaction vessel, allowed to melt into the solution. Once the amethocaine base had fully dissolved the solution was stirred thoroughly and then allowed to cool to room temperature to yield a pourable amethocaine gel.

### Stage 2: Film Formation

A mould was constructed, consisting of a glass plate upon which was placed a release liner releasing surface upwards. An example of a suitable release liner is a fluorinated, non-woven polyester commercially available from 3M Company as Type 9747. This was wetted slightly with water in order to help the liner adhere to the glass plate.

An area (of eg 12 cm by 4 cm (i.e. to yield 4 patches, each 4 cm by 3 cm) was surrounded by a plastic template 3 mm in depth. This template was glued to the glass plate.

The gel of, Stage 1, (10g) was then poured into the centre of the mould, spread out evenly and the whole placed under vacuum to remove air bubbles. The de-aerated gel was allowed to dry in air under ambient conditions or, alternatively, in a moving air stream at 60°C in a drying tunnel.

As an alternative to this procedure, the gel may be directly knife or roller-coated onto the release liner before drying. It will be apparent to those skilled in the art that film formation may thus be achieved by the application of either a batch or continuous process. The film layer produced has sufficient integrity to be peeled off the release liner and rewound if required. The thickness of this layer was between 30 and 300 µm, but most preferably between 50 and 100 µm.

The film was analysed:

| | |
|---|---|
| Gantrez (Trade Mark) | 37% w/w |
| Natrosol (Trade Mark) | 27% w/w |
| Glycerin | 18% w/w |
| Amethocaine | 18% w/w |

### Stage 3: Patch Construction

The dry amethocaine film of Stage 1 was attached to a suitable transfer adhesive such as the acrylate material commercially available in sheet form from 3M Company as Co-Tran Pharmaceutical Grade Transfer Adhesive No. 9871. Alternatively, the transfer adhesive may be knife or roller coated onto the backing layer or onto one side of the film itself.

Once evenly attached the remaining release layer of the transfer adhesive was removed and a metallised foil backing layer attached. An example of a suitable backing layer is a metallised, non-woven polyester foil commercially available from 3M Company as Scotchpak Type 1109. A suitable uncoated strip of the backing layer may be left as a peel strip, typically 5 mm in width.

The patch may be cut to any suitable size, depending on the intended clinical application, and may be individually packaged in a heat-sealable foil with a drug-impenetrable inner liner.

### Example 2

An example of an integrated, water-activated amethocaine patch system prepared according to Example 1 was tested for its percutaneous anaesthetic efficiency according to the pinprick method described in Woolfson, A.D., McCafferty, D.F., McClelland, K.H. & Boston, V. Concentration-response analysis of percutaneous local anaesthetic formulations. British Journal of Anaesthesia, volume 61, pages 589-592 (1988). Eighteen adult volunteers aged between 19 and 33 years of age applied the patch to an area of healthy, intact skin on the forearm, at or near the anterior cubital fossa which had previously been wetted. The patch was applied for 30 minutes in each case, after which it was removed, the treated area delineated and assessed for anaesthesia. All volunteers reported full anaesthesia to pinprick challenge with a mean onset time of 44 minutes and a standard deviation of 6.7 minutes.

## Claims

1. An amethocaine composition in which the amethocaine is in a crystalline free base form and is dispersed in a water activatable adhesive matrix capable of adhering to wet intact healthy human skin, which comprises at least one film forming adhesive polymer, and the composition is provided with a water impermeable backing layer laminated to the amethocaine composition by means of an intermediate transfer adhesive, **characterised in that**
a) the polymer is a copolymer of maleic anhydride and methyl vinyl ether, and
b) the intermediate transfer adhesive is a pressure sensitive adhesive.

2. An amethocaine composition according to claim 1 wherein the adhesive polymer is in a concentration less than that which would retard drug release from a water-activated composition.

3. An amethocaine composition according to claim 2 wherein the film forming polymer also comprises one or more substantially non water activatable adhesive polymer components.

4. An amethocaine composition according to claim 3 wherein the non-water activatable polymer components are selected from hydroxyethylcellulose and sodium carboxymethylcellulose.

5. An amethocaine composition according to claim 1 comprising a plasticiser.

6. An amethocaine composition according to claim 5 wherein the plasticiser is glycerine.

7. An amethocaine composition according to claim 1 wherein the amethocaine base content is from 0.5% to 5% w/w.

8. An amethocaine composition according to any one of the preceding claims wherein the water activatable adhesive matrix comprises a maleic anhydride and methyl vinyl ether copolymer and hydroxyethylcellulose.

9. A method of manufacturing an amethocaine composition according to claim 1 which comprises;
adjusting the pH of an aqueous dispersion of a water activatable adhesive copolymer of maleic anhydride and methyl vinyl ether to pH greater than 7;
adding amethocaine free base to the buffered dispersion;
drying the amethocaine composition and
laminating a backing layer to the amethocaine composition by means of a pressure sensitive intermediate transfer adhesive.

10. The use of amethocaine in the manufacture of an amethocaine composition according to claim 1.

## Patentansprüche

1. Eine Amethocain-Zusammensetzung, in der das Amethocain eine kristallinfreie Basenform aufweist und in einer durch Wasser aktivierbaren Klebematrix, die an nasser, intakter, gesunder Menschenhaut ankleben kann, dispergiert ist, welche mindestens ein filmbildendes Klebepolymer beinhaltet, und wobei die Zusammensetzung mit einer Wasser undurchlässigen Stützschicht versehen ist, die an die Amethocain-Zusammensetzung durch einen intermediären Übertragungskleber laminiert ist, **dadurch gekennzeichnet, dass**:
a) das Polymer ein Copolymer aus Maleinsäureanhydrid und Methylvinylether ist, und
b) der intermediäre Übertragungskleber ein druckempfindlicher Kleber ist.

2. Amethocain-Zusammensetzung gemäß Anspruch 1, wobei das Klebepolymer eine geringere Konzentration aufweist als die, die Freisetzung von Medikamenten aus einer durch Wasser aktivierten Zusammensetzung verzögern würde.

3. Amethocain-Zusammensetzung gemäß Anspruch 2, wobei das filmbildende Polymer auch eine oder mehrere im Wesentlichen nicht durch Wasser aktivierbare Klebepolymer-Komponenten beinhaltet.

4. Amethocain-Zusammensetzung gemäß Anspruch 3, wobei die nicht durch Wasser aktivierbaren Polymer-Komponenten aus Hydroxyethylcellulose und Natriumcarboxymethylcellulose ausgewählt sind.

5. Amethocain-Zusammensetzung gemäß Anspruch 1, die einen Weichmacher beinhaltet.

6. Amethocain-Zusammensetzung gemäß Anspruch 5, wobei der Weichmacher Glyzerin ist.

7. Amethocain-Zusammensetzung gemäß Anspruch 1, wobei der Amethocain-Basengehalt zwischen 0,5 % und 5 % Gewicht/Gewicht liegt.

8. Amethocain-Zusammensetzung gemäß einem der vorhergehenden Ansprüche, wobei die durch Wasser aktivierbare Klebematrix ein Maleinsäureanhydrid und Methylvinylether-Copolymer und Hydroxyethylcellulose beinhaltet.

9. Ein Verfahren zur Herstellung einer Amethocain-Zusammensetzung gemäß Anspruch 1, das Folgendes beinhaltet;
Einstellen des pH-Werts einer wässrigen Dispersion von einem durch Wasser aktivierbaren, Klebepolymer aus Maleinsäureanhydrid und Methylvinylether auf einen pH-Wert, der größer als 7 ist;
Zugeben einer Amethocain freien Base zu der gepufferten Dispersion;
Trocknen der Amethocain-Zusammensetzung und
Laminieren einer Stützschicht an die Amethocain-Zusammensetzung durch einen druckempfindlichen intermediären Übertragungskleber.

10. Die Verwendung von Amethocain bei der Herstellung von einer Amethocain-Zusammensetzung gemäß Anspruch 1.

## Revendications

1. Une composition d'améthocaïne dans laquelle l'améthocaïne est sous une forme de base libre cristalline et est dispersée dans une matrice adhésive activable à l'eau capable d'adhérer à de la peau humaine intacte saine et humide, laquelle comporte au moins un polymère adhésif filmogène, et la composition est fournie avec une couche de support imperméable à l'eau laminée à la composition d'améthocaïne au moyen d'un adhésif de transfert intermédiaire, **caractérisée en ce que**
a) le polymère est un copolymère d'anhydride maléique et d'éther méthylvinylique, et
b) l'adhésif de transfert intermédiaire est un adhésif autocollant.

2. Une composition d'améthocaïne selon la revendication 1 dans laquelle le polymère adhésif est dans une concentration inférieure à celle qui retarderait la libération médicamenteuse d'une composition activée à l'eau.

3. Une composition d'améthocaïne selon la revendication 2 dans laquelle le polymère filmogène comporte aussi un ou plusieurs composants polymères adhésifs substantiellement non activables à l'eau.

4. Une composition d'améthocaïne selon la revendication 3 dans laquelle les composants polymères non activables à l'eau sont sélectionnés parmi l'hydroxyéthylcellulose et la carboxyméthylcellulose de sodium.

5. Une composition d'améthocaïne selon la revendication 1 comportant un plastifiant.

6. Une composition d'améthocaïne selon la revendication 5 dans laquelle le plastifiant est la glycérine.

7. Une composition d'améthocaïne selon la revendication 1 dans laquelle la teneur en améthocaïne base va de 0,5 % à 5 % en poids.

8. Une composition d'améthocaïne selon une quelconque des revendications précédentes dans laquelle la matrice adhésive activable à l'eau comporte un copolymère d'anhydride maléique et d'éther méthylvinylique et de l'hydroxyéthylcellulose.

9. Une méthode de fabrication d'une composition d'améthocaïne selon la revendication 1 qui comporte :
ajuster le pH d'une dispersion aqueuse d'un copolymère adhésif activable à l'eau d'anhydride maléique et d'éther méthylvinylique à un pH supérieur à 7 ;
ajouter la base libre d'améthocaïne à la dispersion tamponnée ;
faire sécher la composition d'améthocaïne, et
laminer une couche de support à la composition d'améthocaïne au moyen d'un adhésif de transfert intermédiaire autocollant.

10. L'utilisation d'améthocaïne dans la fabrication d'une composition d'améthocaïne selon la revendication 1.
